# EUROPEAN PATENT APPLICATION

(11) **EP 4 681 720 A1**
(43) Date of publication of application: **21.01.2026**
(21) Application number: 24770962.9
(22) Date of filing: 14.03.2024
(51) Int. Cl.: A61K 35/44, A61K 35/30, A61P 9/00, A61P 25/00, A61P 25/08, A61P 25/16, A61P 25/24, A61P 25/28, C12N 5/071

(54) **DRUG FOR PREVENTING AND/OR TREATING DISEASE OR CONDITION RELATED TO REDUCED CEREBRAL BLOOD FLOW**

(30) Priority: 15.03.2023 JP 2023041269
(71) Applicant: The University of Osaka, Osaka 565-0871 (JP)
(72) Inventor: TAKAKURA Nobuyuki, Suita-shi, Osaka 5650871 (JP); MATSUI Yuichi, Suita-shi, Osaka 5650871 (JP)
(74) Representative: Vossius & Partner Patentanwälte Rechtsanwälte mbB
(86) International application number: PCT/JP2024/009955
(87) International publication number: WO 2024/190859

(57) **Abstract**

The present invention provides a medicament for preventing and/or treating a disease or condition associated with a decrease in brain blood flow, comprising vascular endothelial cells positive for a CD157 cell surface marker, wherein the cells are from a mammal, wherein the medicament is used so that the vascular endothelial cells are delivered into a brain; and a medicament for regenerating brain blood vessels, comprising vascular endothelial cells positive for a CD157 cell surface marker, wherein the cells are from a mammal, wherein the medicament is used so that the vascular endothelial cells are delivered into a brain.

## Description

### TECHNICAL FIELD

The present invention relates to a medicament for preventing and/or treating a disease or condition associated with a decrease in brain blood flow.

### BACKGROUND ART

The brain represents only 2% of the body weight, but its blood flow volume covers 20% of the cardiac output. The brain blood flow largely contributes to the maintenance of biological activity. A slow and sustained decrease in brain blood flow is termed chronic cerebral hypoperfusion. Chronic cerebral hypoperfusion induces damage to the blood-brain barrier or activates neurodegeneration-inducing molecules or cytotoxicity cascade to serve as a causative factor for white matter lesions (Non-Patent Literatures 1 and 2).

White matter lesions are associated not only with a decrease in cognitive functions but also with depression, hypokinesia, and other dysfunctions, and may lead to acute ischemic stroke (Non-Patent Literature 3). An improvement in chronic cerebral hypoperfusion may lead to a reduction in the risk of these disorders, but currently, no definite therapeutic strategy is available. Non-specific therapies have been attempted, focusing on risk factor management, such as preventive or therapeutic treatment for lifestyle-related diseases, including hypertension. Several studies on therapies for chronic cerebral hypoperfusion have been reported, including pharmacological approaches such as minocycline, cilostazol, or edaravone, rehabilitation approaches such as brain reperfusion rehabilitation therapy, and transplantation approaches such as transplantation of human umbilical cord-derived mesenchymal stem cells. However, there is no clinically widely applicable therapy for chronic cerebral hypoperfusion.

The inventors have reported that CD157-positive vascular endothelial cells are a candidate for vascular endothelial stem cells and are capable of reconstructing functional blood vessels in ischemic liver and lower extremities (Patent Literature 1 and Non-Patent Literature 4). CD157-positive vascular endothelial cells make up a few percentage of the total number of vascular endothelial cells. However, there have been no reports on the impact of the transplantation of CD157-positive vascular endothelial cells with stem cell capacity on an ischemic brain in chronic cerebral hypoperfusion.

### CITATION LIST

### PATENT LITERATURE

Patent Literature 1: WO 2019/098264

### NON-PATENT LITERATURE

Non-Patent Literature 1: Joutel A and Chabriat H, Pathogenesis of white matter changes in cerebral small vessel diseases: beyond vessel-intrinsic mechanisms. Clin Sci (Lond). 2017;131(8):635-651.

Non-Patent Literature 2: Rajeev V et al., Pathophysiology of blood brain barrier dysfunction during chronic cerebral hypoperfusion in vascular cognitive impairment. Theranostics. 2022;12(4):1639-1658.

Non-Patent Literature 3: Pantoni L, Cerebral small vessel disease: from pathogenesis and clinical characteristics to therapeutic challenges. Lancet Neurol. 2010;9(7):689-701.

Non-Patent Literature 4: Wakabayashi T et al., CD157 marks tissue-resident endothelial stem cells with homeostatic and regenerative properties. Cell Stem Cell. 2018;22(3):384-397.e6.

### SUMMARY OF INVENTION

### TECHNICAL PROBLEM

An object of the present invention is to provide a medicament for preventing and/or treating a disease or condition associated with a decrease in brain blood flow. Another object of the present invention is to provide a medicament for regenerating brain blood vessels.

### SOLUTION TO PROBLEM

The present invention was made to solve the above problems and includes the following.
[1] A medicament for preventing and/or treating a disease or condition associated with a decrease in brain blood flow, comprising vascular endothelial cells positive for a CD157 cell surface marker, wherein the cells are from a mammal, wherein the medicament is used so that the vascular endothelial cells are delivered into a brain.
[2] The medicament according to the above [1], wherein the vascular endothelial cells are vascular endothelial cells isolated from a brain.
[3] The medicament according to the above [1] or [2], wherein the mammal is a human.
[4] The medicament according to any one of the above [1] to [3], wherein the disease associated with a decrease in brain blood flow is vascular dementia, carotid artery stenosis, carotid artery occlusion, intracranial artery stenosis, intracranial artery occlusion, moyamoya disease, fibromuscular dysplasia, multiple sclerosis, acute cerebral ischemic disease, chronic cerebral ischemic disease, acute cerebral inflammatory disease, chronic cerebral inflammatory disease, depression, dizziness (vertigo), deafness, headache, cranial neuralgia, cerebellar ataxia, autism, attention deficit hyperactivity disorder, Parkinson's syndrome, epilepsy, schizophrenia, or aging.
[5] The medicament according to any one of the above [1] to [3], wherein the condition associated with a decrease in brain blood flow is a white matter lesion, cerebral atrophy, or neurofibrillary change.
[6] A medicament for regenerating brain blood vessels, comprising vascular endothelial cells positive for a CD157 cell surface marker, wherein the cells are from a mammal, wherein the medicament is used so that the vascular endothelial cells are delivered into a brain.
[7] The medicament according to the above [6], wherein the vascular endothelial cells are vascular endothelial cells isolated from a brain.
[8] The medicament according to the above [6] or [7], wherein the mammal is a human.

### ADVANTAGEOUS EFFECTS OF INVENTION

The present invention provides a medicament for preventing and/or treating a disease or condition associated with a decrease in brain blood flow, and a medicament for regenerating brain blood vessels.

### BRIEF DESCRIPTION OF DRAWINGS

Fig. 1 shows the localization of vascular endothelial cells in brain blood vessels in mice. (A) shows VE-cadherin-positive cells, (B) shows CD31-positive cells, and (C) shows CD157-positive cells. The scale bar is 50 µm.
Fig. 2 shows the results for isolated brain vascular endothelial cells that were fractionated based on CD157 as a marker and then co-cultured with OP9 stromal cells for 10 days. (A) to (C) show immunostaining visualization of CD31-positive cells after the culture, and (D) shows the measurement results of the vascular area formed from each type of cells.
Fig. 3 shows the measurement result of the percentage of CD157-positive cells in the total vascular endothelial cells in the brain of a mouse chronic cerebral hypoperfusion model over time.
Fig. 4 shows the measurement results of the expression levels of genes involved in a neuroprotective effect in CD157-positive vascular endothelial cells and CD157-negative vascular endothelial cells prepared from the brain of a mouse chronic cerebral hypoperfusion model before bilateral common carotid artery stenosis surgery and 1 week after the surgery. (A) shows the result for VEGF-A, and (B) shows the result for EGF.
Fig. 5 shows the results of the quantification of the expression levels of the BDNF gene in CD157-positive vascular endothelial cells and CD157-negative vascular endothelial cells under steady-state conditions (non-ischemic conditions).
Fig. 6 shows a scheme of a transplantation experiment of brain vascular endothelial cells into the brain of a mouse chronic cerebral hypoperfusion model.
Fig. 7 shows the proliferation of transplanted cells observed by *in vivo* imaging on days 7 and 21 after the transplantation of CD157-positive vascular endothelial cells or CD157-negative vascular endothelial cells isolated from the brain of GFP transgenic mice into the brain of a mouse chronic cerebral hypoperfusion model.
Fig. 8 shows the observation of white matter lesions in brains harvested on day 28 after the transplantation of CD157-positive vascular endothelial cells or CD157-negative vascular endothelial cells isolated from the brain of GFP transgenic mice into the brain of a mouse chronic cerebral hypoperfusion model.
Fig. 9 shows the results of an object recognition test after the transplantation of CD157-positive vascular endothelial cells or CD157-negative vascular endothelial cells isolated from the brain of GFP transgenic mice into the brain of a mouse chronic cerebral hypoperfusion model. (A) shows the results on day 14 after the transplantation, and (B) shows the results on day 28 after the transplantation.
Fig. 10 shows *in vivo* angiography imaging on day 21 after the transplantation of total vascular endothelial cells prepared from the brain, liver or fat of GFP transgenic mice into the brain of a mouse chronic cerebral hypoperfusion model.

### DESCRIPTION OF EMBODIMENTS

The present invention provides a medicament comprising vascular endothelial cells positive for a CD157 cell surface marker, wherein the cells are from a mammal, wherein the medicament is used so that the vascular endothelial cells are delivered into a brain (hereinafter referred to as the "medicament of the present invention").

The vascular endothelial cells contained in the medicament of the present invention are any vascular endothelial cells obtained from a mammal. The mammal is not limited to a particular one, and examples thereof include humans, monkeys, cows, pigs, dogs, mice, rats, rabbits, etc. Preferably, the vascular endothelial cells are from a human. Human vascular endothelial cells are safely applicable to humans.

The vascular endothelial cells can be prepared from any organs or tissues. For example, the vascular endothelial cells can be prepared from liver, retina, brain, heart, skin, muscle (skeletal muscle), lung, kidney, placenta, fat, etc. The vascular endothelial cells used in the medicament of the present invention are preferably vascular endothelial cells prepared from a brain. The vascular endothelial cells are defined herein as cells that are positive for a CD31 cell surface marker and negative for a CD45 cell surface marker. Therefore, for example, the vascular endothelial cells can be prepared by digesting and dispersing an isolated organ or tissue in a commercially available cell dissociation reagent to prepare a cell suspension, staining the cell suspension with an anti-CD31 antibody and an anti-CD45 antibody, and collecting CD31-positive, CD45-negative cells using flow cytometry technology. CD157-positive vascular endothelial cells can be prepared by, for example, digesting and dispersing an isolated organ or tissue in a commercially available cell dissociation reagent to prepare a cell suspension, staining the cell suspension with an anti-CD31 antibody, an anti-CD45 antibody, and an anti-CD157 antibody, and collecting CD31-positive, CD45-negative, CD157-positive cells using flow cytometry technology.

The vascular endothelial cells used in the medicament of the present invention may be vascular endothelial cells in which CD157-positive cells coexist with CD157-negative cells, or may be vascular endothelial cells consisting of only CD157-positive cells. The vascular endothelial cells in which CD157-positive cells coexist with CD157-negative cells may be total vascular endothelial cells prepared from any organs or tissues. The percentage of CD157-positive cells in the vascular endothelial cells in which the CD157-positive cells coexist with the CD157-negative cells may be 5% or more, 10% or more, 20% or more, 30% or more, 40% or more, 50% or more, 60% or more, 70% or more, 80% or more, or 90% or more. Preferred are vascular endothelial cells consisting of only CD157-positive cells, and more preferred are vascular endothelial cells consisting of only CD157-positive cells prepared from a brain.

The medicament of the present invention is suitable for use in the prevention and/or treatment of a disease or condition associated with a decrease in brain blood flow. The medicament of the present invention is also suitable for use as a medicament for the regeneration of brain blood vessels. Examples of the disease associated with a decrease in brain blood flow may include vascular dementia, carotid artery stenosis, carotid artery occlusion, intracranial artery stenosis, intracranial artery occlusion, moyamoya disease, fibromuscular dysplasia, multiple sclerosis, acute cerebral ischemic disease, chronic cerebral ischemic disease, acute cerebral inflammatory disease, chronic cerebral inflammatory disease, depression, dizziness (vertigo), deafness, headache, cranial neuralgia, cerebellar ataxia, autism, attention deficit hyperactivity disorder, Parkinson's syndrome, epilepsy, schizophrenia, aging, and the like. Examples of the condition associated with a decrease in brain blood flow may include a white matter lesion, cerebral atrophy, neurofibrillary changes, and the like.

The medicament of the present invention has been confirmed to have a neuroprotective effect. The neuroprotective effect inhibits the cell death of neurons and a decrease in memory. The neuroprotective effect also inhibits the decrease of motor and cognitive functions. The neuroprotective effect further inhibits the denaturation and cell death of neurons due to any acute and chronic ischemia and inflammation, inhibits the denaturation and cell death of neurons due to neurotoxic chemicals, and inhibits the denaturation and cell death of neurons due to lifestyle-related diseases or aging.

The medicament of the present invention can be administered to a living body in the form of a cell suspension of vascular endothelial cells containing CD157-positive cells suspended in an appropriate solution that can be administered to a living body. Examples of the solution that can be administered to a living body include physiological saline, PBS (phosphate-buffered saline), and other physiological salt solutions. The preparation of the vascular endothelial cells is typically performed immediately before administration, but the vascular endothelial cells may be cryopreserved and prepared at the time of use. The medicament of the present invention is used so that CD157-positive vascular endothelial cells are delivered into a brain. The route of administration may be any route of administration that allows for the delivery of CD157-positive vascular endothelial cells into a brain. The route of administration may be, for example, direct administration into a brain, intravenous administration close to a brain, intrathecal administration, etc.

The dosage may vary with the age, body weight, or the like of a patient, and cannot be definitively determined, but a physician can determine an appropriate dosage depending on the circumstances. For example, 1 to 1 × 10⁹ cells may be administered per dose. The frequency of doses can be selected as appropriate from the range of once a day to once a week. The dose and the frequency of doses can be increased or reduced as appropriate for the patient.

The present invention further includes the following.
[A] A medicament for preventing and/or treating a disease or condition associated with a decrease in brain blood flow, comprising administering vascular endothelial cells positive for a CD157 cell surface marker so that the vascular endothelial cells are delivered into a brain of a subject, wherein the cells are from a mammal.
[B] A method for regenerating brain blood vessels, comprising administering vascular endothelial cells positive for a CD157 cell surface marker so that the vascular endothelial cells are delivered into a brain of a subject, wherein the cells are from a mammal.
[C] The method according to the above [A] or [B], wherein the vascular endothelial cells are vascular endothelial cells isolated from a brain.
[D] A mammalian vascular endothelial cell positive for a CD157 cell surface marker for use in prevention and/or treatment of a disease or condition associated with a decrease in brain blood flow.
[E] A mammalian vascular endothelial cell positive for a CD157 cell surface marker for use in regeneration of brain blood vessels.
[F] The mammalian vascular endothelial cell positive for a CD157 cell surface marker for use according to the above [D] or [E], wherein the vascular endothelial cell is a vascular endothelial cell isolated from a brain.
[G] Use of mammalian vascular endothelial cells positive for a CD157 cell surface marker in production of a medicament for preventing and/or treating a disease or condition associated with a decrease in brain blood flow.
[H] Use of mammalian vascular endothelial cells positive for a CD157 cell surface marker in production of a medicament for regenerating brain blood vessels.
[I] Use according to the above [D] or [E], wherein the vascular endothelial cells are vascular endothelial cells isolated from a brain.

### EXAMPLES

The present invention will be described in more detail below with reference to Examples, but the present invention is not limited thereto.

### Example 1: Preparation and analysis of CD157-positive vascular endothelial cells from brain

### 1-1 Confirmation of localization of CD157-positive vascular endothelial cells in brain blood vessels

C57BL/6 mice (male at 8 to 10 weeks old) were euthanized, and fixed by perfusion of 4% paraformaldehyde. The brains were harvested and immersion-fixed. After fixation, the brains were embedded in Tissue-Tek O.C.T. Compound (trade name, Sakura Finetek Japan) to prepare frozen blocks, and the whole brains were sectioned into 20 µm-thick slices. Immunostaining was performed by a conventional method, and the localization of CD157-positive cells was observed under a confocal microscope (TCS SP5, Leica). A rat anti-VE-cadherin antibody (monoclonal antibody, BioLegend), an Armenian hamster anti-CD31 antibody (monoclonal antibody, Merck Millipore), and a PE-conjugated mouse anti-CD157 antibody (monoclonal antibody, BioLegend) were used as primary antibodies. An Alexa Fluor 488-conjugated goat anti-rat IgG antibody (Thermo Fisher) or an Alexa Fluor 647-conjugated goat anti-Armenian hamster IgG antibody (Jackson ImmunoResearch Laboratories) was used as a secondary antibody.

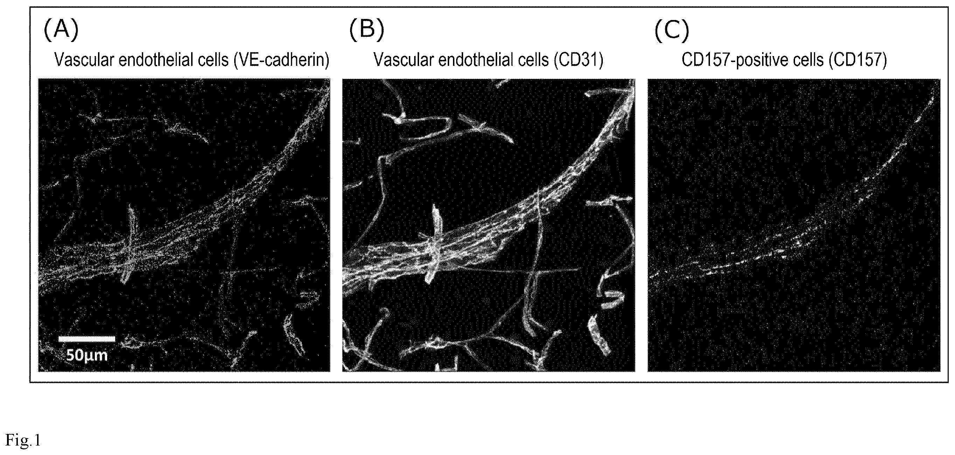

The results are shown in Fig. 1. Immunostaining images show (A) VE-cadherin-positive vascular endothelial cells, (B) CD31-positive vascular endothelial cells, and (C) CD157-positive cells. CD157-positive cells were observed among endothelial cells of blood vessels with a relatively large diameter and localized as a cluster in the brain.

### 1-2 Culture of brain vascular endothelial cells

C57BL/6 mice (male at 5 to 6 weeks old) were euthanized, and the brains were harvested. The brains were enzymatically treated with dispase II (Thermo Fisher Scientific), collagenase (Wako) and Type II collagenase (Worthington Biochemical) to prepare single cell suspensions. Cell surface antigen staining was performed using an anti-CD31 antibody (BD Biosciences), an anti-CD45 antibody (BD Biosciences) and an anti-CD157 antibody (BioLegend). Analysis and cell sorting were performed on a FACSAria SORP cell sorter (BD). Total vascular endothelial cells (CD31+, CD45-), CD157-positive vascular endothelial cells (CD31+, CD45-, CD157+), and CD157-negative vascular endothelial cells (CD31+, CD45-, CD157-) were isolated. Each type of isolated cells (5,000 cells/well) was seeded with OP9 stromal cells (20,000 cells/well) in each well of 24-well plates and co-cultured for 10 days. The culture medium was RPMI-1640 medium (Sigma-Aldrich) supplemented with 10% FCS, 0.1% 2-mercaptoethanol (Gibco) and 1% penicillin-streptomycin (Sigma-Aldrich). After the completion of the culture, the culture medium was removed, and the cells were fixed and immunostained with an anti-CD31 antibody. An anti-CD31 antibody (BD Biosciences) was used as a primary antibody, and a biotin-conjugated anti-rat IgG antibody (Agilent Technologies) was used as a secondary antibody. ABC kit (Vector Laboratories) was used following the instruction manual. Cells were observed at a low magnification using a digital camera (PowerShot SX50, Canon) and at a high magnification using an optical microscope (DMi8, Leica). The vascular area was measured for each group (n = 3).

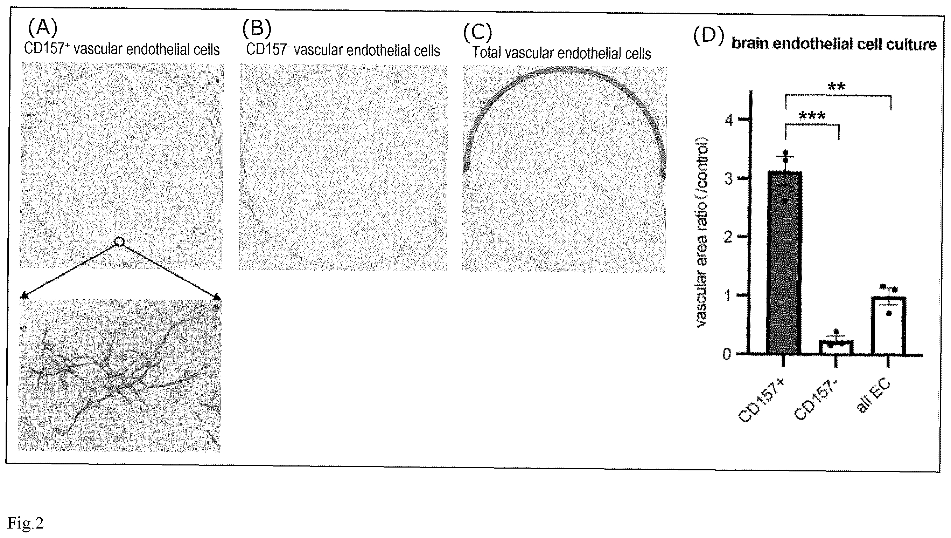

The results are shown in Fig. 2. Immunostaining images show (A) CD157-positive vascular endothelial cells, (B) CD157-negative vascular endothelial cells, and (C) total vascular endothelial cells after each culture. (D) shows the measurement results of the vascular area formed in each culture. In the culture of CD157-positive vascular endothelial cells, a large vascular endothelial cell colony was formed from a single cell, and the vascular area formed from the cell was significantly larger than that formed in the culture of the total vascular endothelial cells. In other words, the results demonstrate that CD157-positive vascular endothelial cells have significantly higher proliferation capacity than CD157-negative endothelial cells.

### Example 2: Analysis of brain vascular endothelial cells under chronic cerebral hypoperfusion conditions

### 2-1 Preparation of mouse chronic cerebral hypoperfusion model

Bilateral common carotid artery stenosis was created in C57BL/6 mice (male at 8 to 10 weeks old) to induce chronic cerebral ischemia. The stenosis procedure was performed by incising the animal's skin along the midline of the neck to expose both common carotid arteries, and wrapping a microcoil (internal diameter 0.18 mm, pitch 0.5 mm, length 2.5 mm) around the arteries. Mouse chronic cerebral hypoperfusion model animals prepared in this manner show an about 70% decrease in brain blood flow compared to that before the surgery, generate white matter lesions, and develop higher brain dysfunction including a decrease in memory, as previously described (Shibata M. et al., Stroke, 2004, 35(11):2598-2603; Nishio K. et al., Stroke, 2010, 41(6):1278-1284).

### 2-2 Measurement of percentage of CD157-positive cells in total vascular endothelial cells

To determine changes in the percentage of CD157-positive vascular endothelial cells in the total vascular endothelial cells (CD31+, CD45-) in the brain of the mouse chronic cerebral hypoperfusion model over time, the brains were harvested before the stenosis surgery and 1, 2, 3 and 4 weeks after the surgery. Single cell suspensions were prepared in the same manner as in Example 1, cell surface antigens were stained, and analysis and cell sorting were performed.

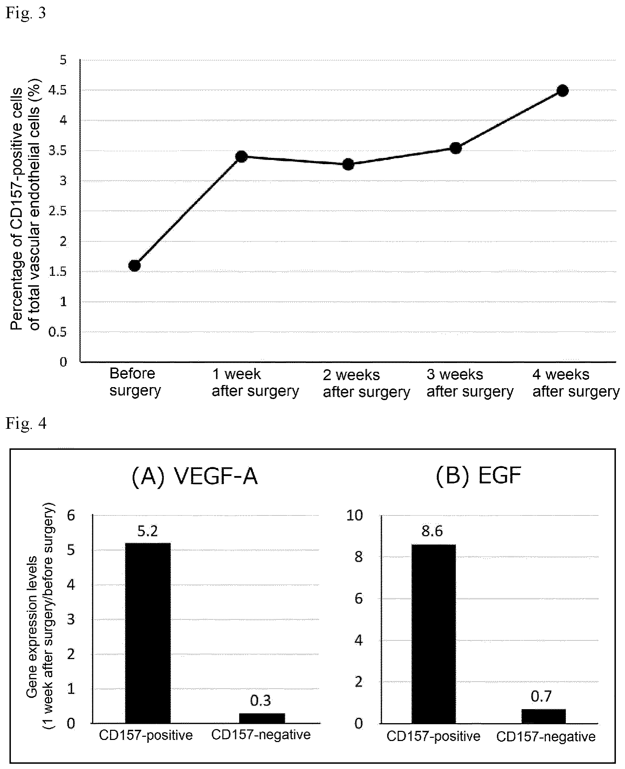

The results are shown in Fig. 3. The percentage of CD157-positive vascular endothelial cells in the brain increased under chronic cerebral hypoperfusion conditions over time, reaching an about 3-fold higher level (4.50%) 4 weeks after the surgery compared to that before the surgery (1.62%). This result demonstrates that a hypoxic state may function as a stimulus that induces the self-renewal of CD157-positive vascular endothelial cells, which may self-induce vascular proliferation via the expansion of vascular endothelial stem cells as a way of selfdefense against a brain disorder.

### 2-3 Analysis of gene expression changes

The gene expression levels in vascular endothelial cells under chronic cerebral hypoperfusion conditions were compared with the gene expression levels under steady-state conditions (non-ischemic conditions). To this end, the brains were harvested from the mouse chronic cerebral hypoperfusion model before the stenosis surgery and 1 week after the surgery. Single cell suspensions were prepared in the same manner as in Example 1, cell surface antigens were stained, and analysis and cell sorting were performed to isolate CD157-positive vascular endothelial cells and CD157-negative vascular endothelial cells. RNAs were extracted from the isolated cells, and cDNAs were synthesized by reverse transcription reaction using RNeasy-plus mini kit (QIAGEN) and PrimeScript RT reagent kit (Takara) following the instruction manuals. RT-qPCR analysis was performed using Platinum SYBR Green qPCR SuperMix-UDC (Invitrogen) on Mx3000p QPCR System (Stratagene). Genes for measurement were VEGF-A (vascular endothelial growth factor-A) and EGF (epidermal growth factor), which are known to have a neuroprotective effect. The following primers were used:
mouse VEGF-A-Fw
   GCCAGCACATAGGAGAGATG (SEQ ID NO: 1),
mouse VEGF-A-Rv
   AAATGCTTTCTCCGCTCTGA (SEQ ID NO:2),
mouse EGF-Fw
   GCCGGCAGATGGGAATGGTT (SEQ ID NO:3), and
mouse EGF-Rv
   GTCTTTCTCGCTGGGACCCA (SEQ ID NO:4).

The results are shown in Fig. 4. (A) shows the result for VEGF-A, and (B) shows the result for EGF. The gene expression levels of VEGF-A and EGF in the CD157-positive vascular endothelial cells significantly increased 1 week after the stenosis surgery compared with those before the surgery. The expression levels of VEGF-A and EGF in the CD157-positive vascular endothelial cells were significantly higher than those in the CD157-negative endothelial cells. These results suggest that CD157-positive vascular endothelial cells residing in the brain may exhibit a potent neuroprotective effect under chronic cerebral hypoperfusion conditions. VEGF-A also has the ability to induce angiogenesis, and hence may also have the ability to induce vascular regeneration in pre-existing blood vessels.

### 2-4 Analysis of expression of BDNF (brain-derived neurotrophic factor) gene under steady-state conditions

The expression levels of BDNF in CD157-positive endothelial cells and CD157-negative endothelial cells were measured by RT-qPCR using the preoperative samples prepared in the above 2-3. The following primers were used:
mouse BDNF-Fw
   GCGGACCCATGGGACTCT (SEQ ID NO: 5), and
mouse BDNF-Rv
   CTGCTGCTGTAGTGACCGA (SEQ ID NO: 6).

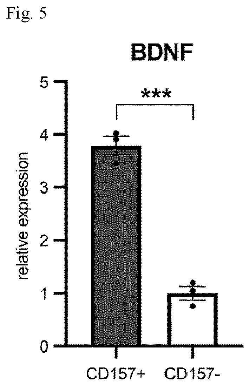

The results are shown in Fig. 5. The results reveal that BDNF is strongly expressed in CD157-positive vascular endothelial cells compared with CD157-negative vascular endothelial cells under steady-state conditions (non-ischemic conditions). The results suggest that CD157-positive endothelial cells may exhibit a potent neuroprotective effect when used in transplantation.

### Example 3: Transplantation of vascular endothelial cells into brain of mouse chronic cerebral hypoperfusion model (1)

### 3-1 Experimental Method

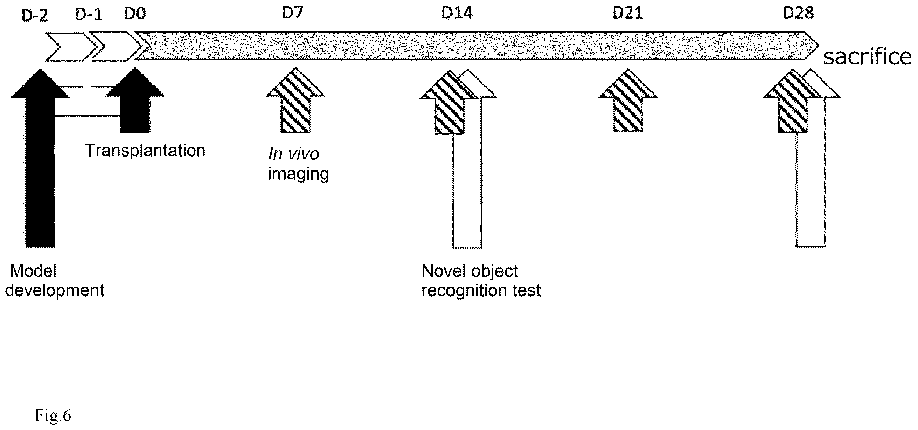

Fig. 6 shows the scheme of the transplantation experiment.

### (1) Transplantation method

Two days before transplantation, a mouse chronic cerebral hypoperfusion model was prepared by the same procedure as in Example 2. One day before transplantation, 5- to 6-week-old C57BL/6-Tg (CAG-EGFP) mice (GFP transgenic mice, hereinafter referred to as "GFP mice") were euthanized, and the brains were harvested to prepare single cell suspensions in the same manner as in Example 1. Cell surface antigens were stained, and cell sorting was performed to isolate CD157-positive vascular endothelial cells and CD157-negative vascular endothelial cells. The cells were cultured with shaking in HuMedia-EB2 (Kurabo) supplemented with HuMedia-EG (Kurabo) and 2% B-27 supplement (Thermo Fisher Scientific) overnight. On the transplantation day, 300 µL of 10% glycerol (Chugai Pharmaceutical) was intraperitoneally administered to prevent cerebral edema during surgery. Then, a hole was made in the cranial bone of the model mouse, and the dura was exposed and removed to expose the right cerebral cortex. Stereotactic injection of 2 × 10⁴ cells of the CD157-positive vascular endothelial cells or the CD157-negative vascular endothelial cells was performed at a position 3 mm lateral to and 1 mm posterior to the bregma at a depth of 60 µm. The transplantation site was filled with an artificial cerebrospinal fluid, and closed with a 5-mm silicone-coated circular glass cover using a medical adhesive.

### (2) In vivo imaging

*In vivo* imaging was performed using a confocal/two-photon excitation microscope (TCS SP8, Leica) on days 7, 14, 21 and 28 after the transplantation to observe the proliferation of the transplanted endothelial cells and measure the area of the region in which the transplanted vascular endothelial cells contributed to angiogenesis.

### (3) Observation of white matter lesions

Chronic cerebral ischemia is known to develop white matter lesions over time, including the loss of myelin and the proliferation of activated astrocytes, which may be responsible for a decrease in cognitive functions and others. The proliferation of activated astrocytes can be examined by GFAP (glial fibrillary acidic protein) staining (Wakita H. et al., Acta Neuropathol, 1994).

The mice were euthanized on day 28 after the transplantation, and frozen blocks of the brains were prepared by the same procedure as in Example 1. The region from 1.5 mm anterior to 2.5 mm posterior to the bregma was cut out, and sectioned into 20 µm-thick slices. Immunostaining was performed by a conventional method using a rabbit anti-GFAP antibody (polyclonal antibody, Sigma-Aldrich) as a primary antibody and Alexa Fluor 546-conjugated goat anti-rabbit IgG (Thermo Fisher Scientific) as a secondary antibody. The medial region of the corpus callosum was observed using a confocal microscope (TCS SP5, Leica) to examine white matter lesions. The volume of a GFPA-positive area and the intensity of GFAP-positive fluorescence were measured using a Volocity 3D imaging and analysis software (Perkin Elmer). The intensity of GFAP-positive fluorescence reflects the number of activated astrocytes, indicating the severity of white matter lesions.

### (4) Novel object recognition test (NOR)

A novel object recognition test was performed on days 14 and 28 after the transplantation to evaluate the memory of the mice. On one day before the test, individual mice were placed into a black plastic open-field box of 30 cm × 30 cm × 30 cm and acclimated for 15 minutes. On the test day, two light-blue wooden triangle blocks A-A (5 cm × 3 cm × height 3 cm) were symmetrically placed 10 cm away from the nearest wall. The mouse was placed at an equal distance from the two objects and allowed to explore the box for 15 minutes. Ninety minutes later, one object of the configuration A-A was replaced with a novel object (a red wooden cylinder of 3 cm in height and 3 cm in diameter). The mouse was placed at an equal distance from the two objects and allowed to explore the box for 15 minutes. The time spent exploring the familiar objects (F) and the time spent exploring the novel object (N) were recorded, and the novelty score [N/(N+F)] was calculated. Exploratory behavior was defined as the animal sniffing or touching any object at a distance of 1 cm from the snout, and sitting on the object was not considered exploratory behavior. The device and the objects were cleaned with 70% alcohol between experiments to avoid providing any smell stimulus.

### 3-2 Results

### (1) In vivo imaging

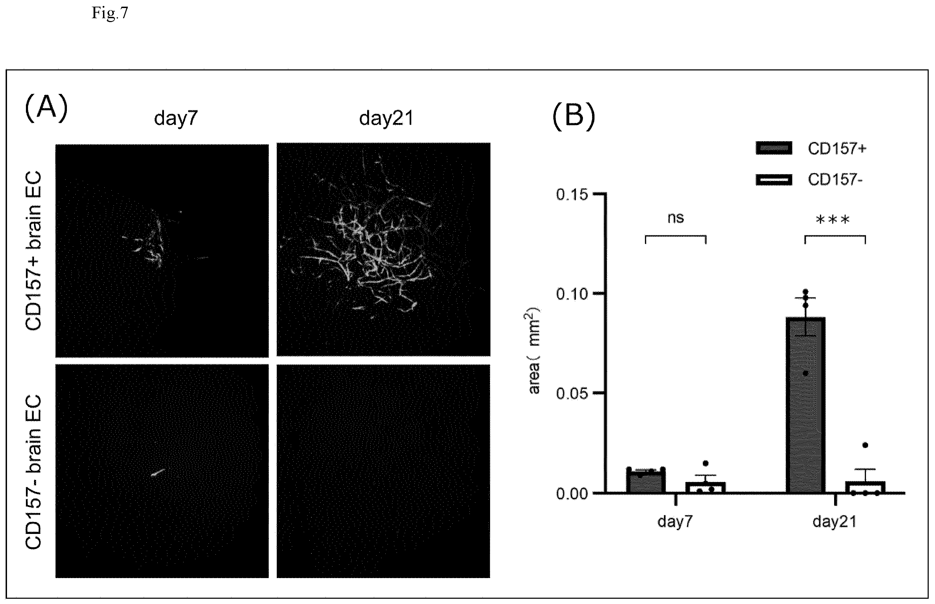

Fig. 7 shows the observation of the transplanted vascular endothelial cells from the GFP mice. (A) shows *in vivo* images on day 7 (left) and day 21 (right) after the transplantation. The upper panels are images of the brains transplanted with the CD157-positive vascular endothelial cells, and the lower panels are images of the brains transplanted with the CD157-negative vascular endothelial cells. The white part in the figures is GFP-positive cells. (B) shows the measured vascular area determined from the obtained images. As shown in (A), the CD157-positive vascular endothelial cells from the GFP mouse cells established a large vascular network in the brain on day 21 after the transplantation, whereas the CD157-negative vascular endothelial cells did not establish a vascular network. As shown in (B), no significant difference was observed between the CD157-positive vascular endothelial cell transplantation group (n = 4) and the CD157-negative endothelial cell transplantation group (n = 4) on day 7 after the transplantation. However, the vascular area was significantly larger in the CD157-positive vascular endothelial cell group on day 21 after the transplantation. The results confirm that CD157-positive vascular endothelial cells exhibit high proliferation capacity not only *in vitro* (Example 1) but also *in vivo* (in the brain).

### (2) Observation of white matter lesions

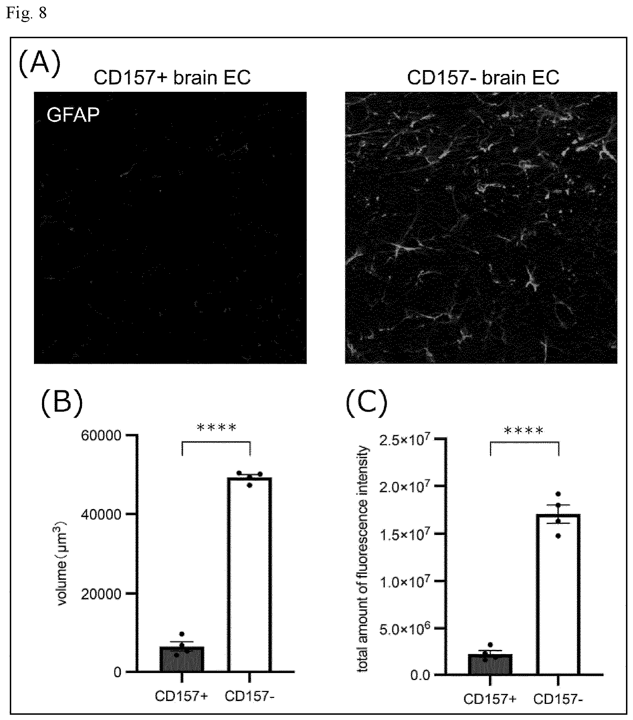

The results are shown in Fig. 8. (A) shows the immunostaining results. The left panel shows the brain transplanted with the CD157-positive vascular endothelial cells, and the right panel shows the brain transplanted with the CD157-negative vascular endothelial cells. (B) shows the measurement results of the volume of the GFAP-positive area. (C) shows the measurement result of the total intensity of GFAP-positive fluorescence. As shown in (A), almost no GFAP-positive cells were detected in the brain transplanted with the CD157-positive vascular endothelial cells, whereas a large number of GFAP-positive cells were detected in the whole region in the brain transplanted with the CD157-negative vascular endothelial cells. As shown in (B), the volume of white matter lesions was 0.65 × 10⁴ µm³ in the CD157-positive vascular endothelial cell transplantation group (n = 4), whereas the volume of white matter lesions was 4.93 × 10⁴ µm³ in the CD157-negative vascular endothelial cell transplantation group (n = 4). As shown in (C), the total intensity of the fluorescence was 0.22 × 10⁷ in the CD157-positive vascular endothelial cell transplantation group (n = 4), whereas the total intensity of the fluorescence was 1.70 × 10⁷ in the CD157-negative vascular endothelial cell transplantation group (n = 4). The results confirm that transplantation of CD157-positive vascular endothelial cells significantly alleviates white matter lesions.

### (3) Novel object recognition test

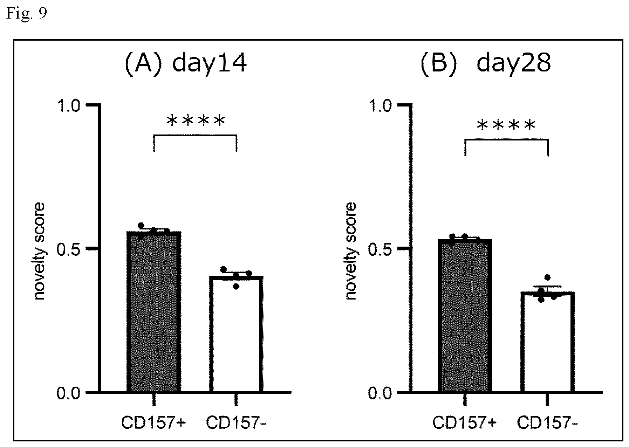

The results are shown in Fig. 9. (A) shows the results on day 14 after the transplantation, and (B) shows the results on day 28 after the transplantation. On day 14 after the transplantation (day 14), the novelty score of the CD157-positive vascular endothelial cell transplantation group (n = 4) was 0.56, whereas the novelty score of the CD157-negative vascular endothelial cell transplantation group (n = 4) was 0.41, indicating a significant difference between the two groups. On day 28 after the transplantation (day 28), the novelty score of the CD157-positive vascular endothelial cell transplantation group (n = 4) was 0.53, whereas the novelty score of the CD157-negative vascular endothelial cell transplantation group (n = 4) was 0.35, indicating a significant difference between the two groups. The decrease in the score from day 14 to day 28 was small in the CD157-positive vascular endothelial cell transplantation group, indicating that more memory was maintained due to the transplantation of the CD157-positive vascular endothelial cells. The results reveal that the blood vessels formed by CD157-positive vascular endothelial cells have a potent protective effect on cranial nerves and have a high effect on dementia.

### Example 4: Transplantation of vascular endothelial cells into brain of mouse chronic cerebral hypoperfusion model (2)

Total vascular endothelial cells were transplanted into the brain of the mouse chronic cerebral hypoperfusion model in the same manner as in Example 3, except that total vascular endothelial cells (CD31+, CD45-) prepared from each of the brain, liver, and fat of 5- to 6-week-old GFP mice were used. To examine the relationship between the donor blood vessels and the recipient blood vessels, angiography was performed on day 21 after the transplantation. Angiography was carried out by administering 100 µL of a fluorescent reagent (AngioSPARK 680, PerkinElmer) via the retro-orbital plexus and observing the blood vessels by *in vivo* imaging using a confocal/two-photon excitation microscope (TCS SP8, Leica).

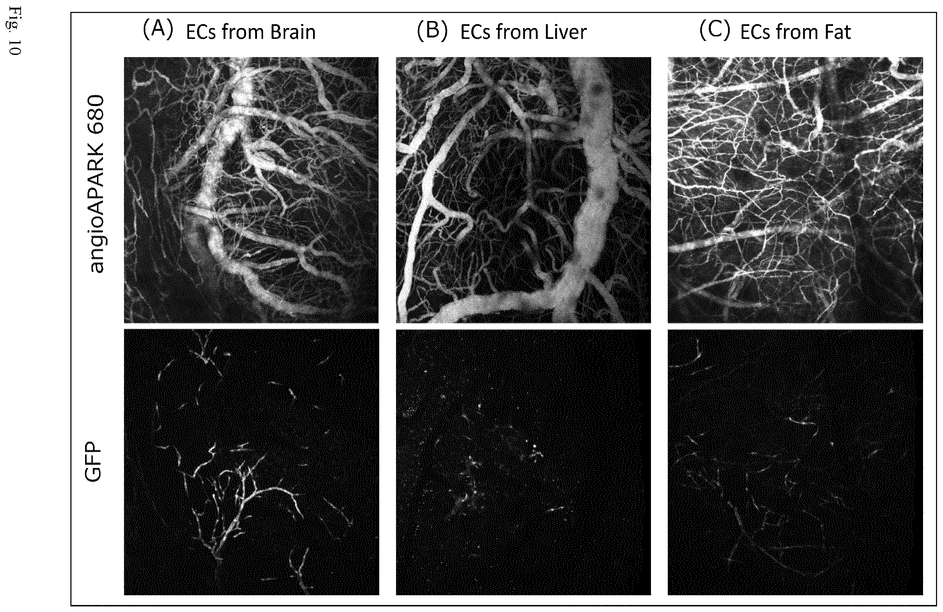

The results are shown in Fig. 10. Images of blood vessels in the brain of the mouse chronic cerebral hypoperfusion model transplanted with the vascular endothelial cells from (A) brain, (B) liver or (C) fat. The upper panels show the blood vessels visualized with a contrast agent, and the lower panels show GFP-positive blood vessels. The blood vessels formed from the transplanted vascular endothelial cells are GFP positive. The continuity between the donor blood vessels and the recipient blood vessels was observed regardless of the organ serving as the source of the transplanted vascular endothelial cells, but was most frequently observed when the brain vascular endothelial cells were transplanted. The results indicate that brain vascular endothelial cells are the most excellent as vascular endothelial cells for transplantation into a brain.

The present invention is not limited to each of the embodiments or Examples as described above, and various modifications are possible within the scope of the claims. Embodiments obtainable by appropriately combining the technical means disclosed in the different embodiments of the present invention are also included in the technical scope of the present invention. The contents of the scientific literature and the patent literature cited herein are hereby incorporated by reference in their entirety.

## Claims

1. A medicament for preventing and/or treating a disease or condition associated with a decrease in brain blood flow, comprising vascular endothelial cells positive for a CD157 cell surface marker, wherein the cells are from a mammal, wherein the medicament is used so that the vascular endothelial cells are delivered into a brain.

2. The medicament according to claim 1, wherein the vascular endothelial cells are vascular endothelial cells isolated from a brain.

3. The medicament according to claim 1, wherein the mammal is a human.

4. The medicament according to any one of claims 1 to 3, wherein the disease associated with a decrease in brain blood flow is vascular dementia, carotid artery stenosis, carotid artery occlusion, intracranial artery stenosis, intracranial artery occlusion, moyamoya disease, fibromuscular dysplasia, multiple sclerosis, acute cerebral ischemic disease, chronic cerebral ischemic disease, acute cerebral inflammatory disease, chronic cerebral inflammatory disease, depression, dizziness (vertigo), deafness, headache, cranial neuralgia, cerebellar ataxia, autism, attention deficit hyperactivity disorder, Parkinson's syndrome, epilepsy, schizophrenia, or aging.

5. The medicament according to any one of claims 1 to 3, wherein the condition associated with a decrease in brain blood flow is a white matter lesion, cerebral atrophy, or neurofibrillary change.

6. A medicament for regenerating brain blood vessels, comprising vascular endothelial cells positive for a CD157 cell surface marker, wherein the cells are from a mammal, wherein the medicament is used so that the vascular endothelial cells are delivered into a brain.

7. The medicament according to claim 6, wherein the vascular endothelial cells are vascular endothelial cells isolated from a brain.

8. The medicament according to claim 6 or 7, wherein the mammal is a human.
